(19) Europäisches Patentamt
European Patent Office
Office européen des brevets

(11) EP 2 481 733 A1

(12) EUROPEAN PATENT APPLICATION

(43) Date of publication:
**01.08.2012 Bulletin 2012/31**

(51) Int Cl.:
***C07D 307/68*** *(2006.01)*

(21) Application number: **11152507.7**

(22) Date of filing: **28.01.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Süd-Chemie AG**
**80333 München (DE)**

(72) Inventors:
- **Richter, Dr. Oliver**
  **82110 Germering (DE)**
- **Franke, Dr. Oliver**
  **81671 München (DE)**

(74) Representative: **HOFFMANN EITLE**
**Patent- und Rechtsanwälte**
**Arabellastraße 4**
**81925 München (DE)**

(54) **Process for manufacturing esters of 2,5-furandicarboxylic acid**

(57) The present invention relates to an improved process for manufacturing of esters of 2,5-furandicarboxylic acid (FDCA-esters) by reacting 2,5-furandicarboxylic acid (FDCA) with one or more alcohols in the presence of a heterogeneous catalyst. The use of the heterogeneous catalyst, which may be a Brönsted or Lewis acid, allows for production of high yields of FDCA esters. Furthermore, the catalyst can conveniently be recycled after completion of the reaction.

EP 2 481 733 A1

**Description**

**Field of the invention**

The present invention relates to an improved process for manufacturing of esters of 2,5-furandicarboxylic acid (FDCA-esters) according to the following general Formula I,.

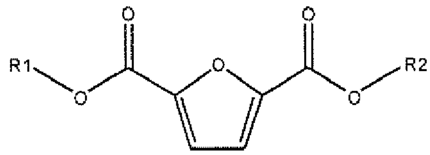

Formula I

wherein R1 is selected from Alkyl, Aryl, H; and wherein R2 is selected from Alkyl, Aryl, and wherein the Alkyl or Aryl moieties may be the same or different and may optionally be substituted..

**Background of the invention**

**Brief description of the prior art**

Esterification of carboxylic acids with alcohols by the use of a homogeneous or heterogeneous Brönsted and/or Lewis-acid catalyst has previously been described (Otera, Nishikido, Esterification: Methods, Reactions, and Applications, 2nd Ed., WILEY-VCH Verlag GmbH & Co. KGaA, Weinheim, 2010, p.6 ff.). Typically, esters of 2,5-furandicarboxylic acid (FDCA-esters) are prepared by acid catalyzed dehydration of galactaric acid (mucic acid) or galactaric acid esters in the presence of an alcohol (e.g. methanol, ethanol, butanol). For this purpose, mineral acids such as HCl, $H_2SO_4$, and HBr or heteropoly acids such as $H_3PW_{12}O_{40}\cdot nH_2O$ are used as catalysts (Taguchi et al., Chemistry Letters Vol. 37, 2008, p.50; Lewkowski, Polish Journal of Chemistry Vol. 75, 2001, p.1943; FR 2723946; US 2673860). For the synthesis route based on galactaric acid, 2,3-furandicarboxylic acid ester is observed as major by-product limiting the yield of the main-product 2,5-furandicarboxylic acid ester to 90 % (FR 2723946). The starting material galactaric acid is usually synthesized by oxidation of galacturonic acid, which is a rare and high pricing feedstock. Therefore, the manufacturing of FDCA-esters on the basis of galactaric acid is additionally limited by economical reasons.

Casanova et al. (Journal of Catalysis, Vol. 265, 2009, p.109) reported an alternative synthesis of FDCA-esters by an oxidative esterification of 5-hydroxymethylfurfural (HMF) using gold on nanoparticulated ceria as the catalyst. Depending on the alcohol used, low catalytic activities were found resulting in large reaction times up to 24 h.

Only one homogenous catalyst is reported so far for the esterification of 2,5-furandicarboxylic acid (FDCA) with an alcohol. Lewkowski (Polish Journal of Chemistry Vol. 75, 2001, p.1943) describes the esterification of FDCA with methanol and ethanol using p-toluene sulfonic acid. However, only moderate yields of 68 % FDCA-dimethyl ester and 73 % FDCA-diethylester, respectively, were achieved by using this method. Additionally, the homogeneous catalyst used has various further drawbacks such as corrosion of the reactor device, no reusability of the catalyst due to separation problems, or environmental impact of waste by salts formed during neutralization.

Various uses for FDCA esters have been described, making the development of efficient production methods necessary. Up to date, the use of FDCA-diesters as a narcotic substance (US 2673860) or as monomer for manufacturing thermoplastic resins by transesterification (JP 2008-291244) is reported.

Problem to be solved

However, a challenge at esterification reactions is the structure of the carboxylic acid and alcohol to be used because these control the reactivity and so the product yields (Kirby, Hydrolysis and formation of esters of organic acids., in: Bamford, Tipper (Ed.), Comprehensive Chemical Kinetics, Vol. 10 Ester Formation, Hydrolysis and Related Reactions, Elsevier, Amsterdam, 1972, p.130 ff.). According to the state of the art (Lewkowski, Polish Journal of Chemistry Vol. 75, 2001, p.1943), esters of FDCA can only be obtained in relatively low yields. Furthermore, the state of the art does not provide a convenient method for catalyst recycling. The problem to be solved by the present invention is therefore the provision of an improved method for esterification of FDCA.

**Brief description of the invention**

The present invention relates to a process for manufacturing esters of 2,5-furandicarboxylic acid by reacting 2,5-furandicarboxylic acid with one or more alcohols in the presence of a heterogeneous catalyst. Preferably, the catalyst used in this process is a Brönsted and/or Lewis acid catalyst. The heterogeneous catalyst is preferably selected from the group consisting of

a) silica-based materials
b) clays
c) aluminas
d) ion exchange resins
e) metal oxides
f) heteropoly acids
g) hydroxyapatite.

Particularly preferred in the process according to the invention is a heterogeneous catalyst which is a silica-based material selected from the group consisting of

a1) amorphous silica-aluminas, preferably with an $Al_2O_3/SiO_2$ ratio from 0.2 to 20, more preferably with an $Al_2O_3/SiO_2$ ratio from 1.0 to 3.0, and most preferably with an $Al_2O_3/SiO_2$ ratio from 2.0 to 2.5;
a2) zeolites, preferably of the type FAU, MOR, MFI, LTA or BEA, and especially preferred of the type FAU with an $SiO_2/Al_2O_3$ratio from 2 to 100; and
a3) mesoporous molecular sieves, preferably a metal-doped mesoporous molecular sieve, and more preferably an aluminium-doped mesoporous molecular sieve type MCM-41 with a Si/Al ratio from 5 to 200.

In this process, the catalyst is preferably in the form of particles, such as preferably in the form of powder.

The reaction is carried out in a reaction chamber/reactor vessel (The terms reaction chamber and reactor vessel are used interchangeably throughout this document). In the process according to the invention, the reaction chamber may optionally be heated. It is favourable that the reaction chamber is heated to a temperature of 100 °C or more, preferably to a temperature of 160 °C or more, more preferably to a temperature of 200 °C to 350 °C, and more preferably to a temperature of 250 °C to 300 °C. The heating occurs preferably for a time period sufficient to carry out the reaction to a satisfying degree, such as, that at least 30 %, preferably at least 50 % and more preferably at least 70 % of the FDCA are esterified at least one carboxyl group, preferably at both hydroxyl groups. Preferred reaction times are 60 min or more, preferably for 60 to 360 min, more preferably for 120 to 300 min. It is understood to be advantageous that the reaction mixture is agitated during part or all of the process described above.

In the process according to the present invention the w/w ratio of heterogeneous catalyst to FDCA is preferably in the range from 100:1 to 1:100, preferably 10:1 to 1:10, and most preferably approximately 1:1. It is further preferred that the initial concentration of FDCA is in the range from 0.1 to 10 wt.-%, preferably 1 to 5 wt.-%. Examples 5 and 6 show different initial amounts of FDCA in the reaction mixture.

Any alcohol or mixture of alcohols may be used which is suitable to obtain an FDCA ester. However, in the process according to the invention, it is preferred that the one or more alcohol is aliphatic alcohol, preferably monovalent aliphatic alcohol. A monovalent alcohol is an alcohol having one OH group. It is particularly preferred that the one or more aliphatic alcohol comprises or comprise one to four carbon atoms, and is or are preferably selected from methanol, ethanol, 1-propanol, 1-butanol and iso-butanol. Preferably one alcohol or a mixture of two alcohols is used, but even more preferably, only one alcohol is used. If only one alcohol is used, it is preferably selected as such that the main product of the process according to the invention is selectable from dimethyl ester, di-ethyl ester, di-propyl ester, di-butyl ester and di-isobutyl ester.

A purge gas is preferably present in the process according to the invention; the purge gas is preferably an inert gas, i.e. a gas that is non-reactive with the educts, products, intermediates and apparatus of the process according to the present invention. Useful inert gases are nitrogen, carbon dioxide, and all noble gases, such as neon and argon and mixtures thereof. Particularly preferred is a purge gas that is or comprises nitrogen gas. One main purpose of the purge gas is removal of water that is formed in the esterification process. Thus, the water may be removed from the purge gas after exit from the reaction chamber, such as by condensation, adsorption. The purge gas is preferably recycled. During the process according to the present invention, the pressure in the reaction chamber can preferably reach a maximal pressure of 1 bar or more, preferably 3 bar or more and more preferably 5 bar or more. Generally, the reaction mixture comprising FDCA, alcohol and a heterogeneous catalyst may be heated in a reactor device to the desired reaction temperature. After the reaction time, the reaction mixture, which may be in the form of a slurry, is cooled down and the heterogeneous catalyst is preferably removed. Subsequently, the formed FDCA-ester can be separated from the remaining alcohol.

**Detailed description of the invention**

The present invention describes the improved esterification of 2,5-furandicarboxylic acid (FDCA) with an alcohol. The present invention resolves the problems of the state of the art described above by using a heterogeneous catalyst. The heterogeneous catalyst according to the invention is preferably highly active and highly selective to FDCA-diesters for the esterification of FDCA. That is, it is suitable to achieve higher yields than the process according to the state of the art. Yield is thereby defined as follows:

$$Y(i) = \frac{n(i)}{n(FDCA, 0)} \cdot 100\%$$

| | |
|---|---|
| Y(i): | molar yield of FDCA-ester i [%] |
| n(FDCA,0): | initial amount of FDCA [mol] |
| n(i): | produced amount of FDCA-ester i [mol]. |

Surprisingly, it was found that the esterification of FDCA with an alcohol is catalyzed by a heterogeneous acid catalyst yielding up to 100 % of the corresponding ester. Further advantages of the process according to the present invention are that typically neither salt impurities (typically in case of using mineral acids), nor sulfur impurities (typically in case of using p-toluene sulfonic acid) are observed by using a heterogeneous catalyst.

Thus, the present invention relates to a process for manufacturing esters of 2,5-furandicarboxylic acid according to formula I:

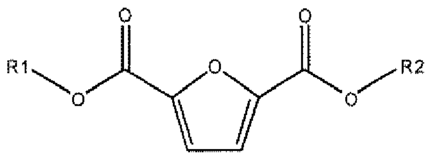

Formula I

by reacting 2,5-furandicarboxylic acid with one or more alcohols in the presence of a heterogeneous catalyst. The embodiments of R1 and R2 of formula I within the meaning of this invention are given below.

The one or more alcohol and the FDCA may be of any source. However, in a preferred embodiment, the alcohol and/or the FDCA can be obtained from biomass. Methods of obtaining alcohols from biomass, such as by fermentation, are well known. Methods of obtaining FDCA from biomass have been described by T. Werpy, G. Petersen: Top Value Added Chemicals from Biomass. Volume I - Results of Screening for Potential Candidates from Sugars and Synthesis Gas. Produced by the Staff at Pacific Northwest National Laboratory (PNNL); National Renewable Energy Laboratory (NREL), Office of Biomass Program (EERE), 2004. Thus, in a particular embodiment, it is possible to couple the esterification process of this invention to production of the one or more alcohol from biomass and/or the production of FDCA from biomass.

Any heterogeneous substance capable of catalyzing the condensation of one or more alcohols with FDCA is a suitable catalyst according to the invention. A heterogeneous substance is to be understood as a substance which is not soluble in the reaction mixture, i.e. any substance which is in solid state at room temperature, such as crystalline or amorphous, and which is preferably in solid state at the maximum reaction temperature during the process according to the invention. Substances with a melting point above 500 °C are particularly preferred.

Preferably, the catalyst used in this process is a Brönsted and/or Lewis acid catalyst. Brönsted and Lewis acids are definitions well known to the person skilled in the art. For reference, see for example: Kolditz, Anorganikum, Deutscher Verlag der Wissenschaften, Berlin, 1970, page 423. Nonlimiting illustrative examples of preferred catalysts are given in example 1.

The heterogeneous catalyst is preferably selected from the group consisting of

a) silica-based materials
b) clays
c) aluminas
d) ion exchange resins
e) metal oxides
f) heteropoly acids
g) hydroxyapatite.

Appropriate heterogeneous catalysts of type b), c), d), e), f) or g) above comprise the following:

b) clays, preferable activated clays, especially preferred acid activated clays
c) aluminas, preferable metal doped aluminas, especially preferred titanium, zirconium and/or cerium doped aluminas
d) ion exchange resins, preferable ion exchange resins with polymer matrix, especially preferred ion exchange resins with polymer matrix and carboxyl, carboxymethyl or sulfone functional groups
e) metal oxides, preferable sulfated and/or phosphated metal oxides, especially preferred sulfated zirconium oxide
f) heteropoly acids, preferable heteropoly acids containing tungsten, molybdenum and/or vanadium as addenda atoms and silicon, phosphorous and/or arsenic as hetero atoms, especially preferred $SiO_2 \cdot 12WO_3 \cdot 26H_2O$, $H_3PW_{12}O_{40} \cdot nH_2O$, $H_3PMo_{12}O_4$, $H_3PMo_6V_6O_{40}$ and $H_5PMo_{10}V_2O_{40}$
g) hydroxyapatite, preferable hydroxyapatite with a Ca/P ratio from 1.50 to 1.70, especially preferred hydroxyapatite with a Ca/P ratio from 1.64 to 1.68

However, even more preferred than the materials of type b), c), d), e), f) or g) above are heterogeneous catalysts which are silica-based materials of type a) above. A silica-based material within the meaning of this definition is any material which comprises the elements silicon and oxygen, the amount of silicon being 10 mol-% or more, in a particular embodiment 20 mol-% or more. Particularly preferred in the process according to the invention is a silica-based material selected from the group consisting of:

a1 amorphous silica-aluminas with an $Al_2O_3/SiO_2$ ratio from 0 to 1000, preferably with an $Al_2O_3/SiO_2$ ratio from 0.2 to 20, more preferably with an $Al_2O_3/SiO_2$ ratio from 1.0 to 3.0, and most preferably with an $Al_2O_3/SiO_2$ ratio from 2.0 to 2.5;
a2) zeolites, preferably of the type FAU, MOR, MFI, LTA or BEA, and especially preferred of the type FAU with an $SiO_2/Al_2O_3$ ratio from 2 to 100; and
a3) mesoporous molecular sieves, preferably a metal-doped mesoporous molecular sieve, and more preferably an aluminium-doped mesoporous molecular sieve type MCM-41 with a Si/Al ratio from 5 to 200.

In this process, the catalyst is preferably in the form of particles, and more preferably in the form of granular particles, and most preferably in the form of powder. The term "particles" is not associated with any particular size limitation. However, "granular particles" are to be understood as particles with a z-average diameter of less than 0.5 cm, preferably less than 0.1 cm, and even more preferably between 0.1 cm and 1 μm. A "powder" is understood as a material of very fine particles which are typically not cemented together. This means that the particles of the powder have a z-average diameter of 500 μm or less, preferably 250 μm or less, more preferably 100 μm or less, and most preferably in the range from 25 μm to 75 μm. The z-average particle size can be measured by any method known to the person skilled in the art, such as by analytical sieving.

However, alternatively a shape-formed catalyst may be used and deposited in the reaction tank. Use of a shape-formed catalyst allows for the use of continuous or semi-continuous operation mode. For this purpose, a shape formed catalyst may be deposited inside the reactor vessel.

The catalysts according to this invention allow obtaining very good yields of FDCA esters. This finding is surprising, because in a previous study, wherein terephthalic acid (TPA) was esterified (Palani et al, J. Mol. Catal. 245 (2006), 101-105) the yields obtained were significantly lower than according to the present invention.

The reaction is carried out in a reaction chamber. A reaction chamber is any chamber suitable for carrying out the reaction, without being limited in size or shape. Criteria for selecting a suitable reaction chamber are known to the person skilled in the art. Suitable reaction chambers especially include the ones disclosed in the examples of this document. The material of the reaction chamber is not limited to a particular material, However, the skilled person will appreciate that the material should be a material which is stable at the reaction conditions, such as at the temperature and pressure which are used during the process, examples and preferred embodiments for such temperatures and pressures are given below.

In the process according to the invention, the reaction chamber may optionally be heated. The purpose of heating is acceleration of the reaction. Example 4 below teaches that heating is indeed beneficial for the yield of FDCA ester obtained. It is favourable that the reaction chamber is heated to a temperature of 100 °C or more, preferably to a temperature of 160 °C or more, more preferably to a temperature of 200 °C to 350 °C, and more preferably to a temperature of 250 °C to 300 °C. Yields obtainable at different temperatures and reaction times are given examples 3 and 4. The heating occurs preferably for a time period sufficient to carry out the reaction to a satisfying degree, such as, that at least 30 %, preferably at least 50 % and more preferably at least 70 % of the FDCA are esterified at at least one carboxyl group, preferably at both carboxyl groups. Preferred reaction times are 60 min or more, preferably for 60 to 360 min, more preferably for 120 to 300 min. Yields obtainable at different temperatures and reaction times are given examples 3 and 4. Hence, in the above-described embodiment of heating the reaction chamber during the reaction, the reaction chamber is a thermostable reaction chamber, i.e. consisting of a material which is physically and chemically stable at the elevated temperature, i.e. which does not disintegrate.

It is understood to be advantageous that the reaction mixture is agitated during part or all of the process described above. Therefore, the reaction mixture is agitated in a preferred embodiment of carrying out this invention. Any means suitable to put all or part of the reaction mixture into motion may be applied. Non-limiting examples include external agitation such as shaking, internal agitation such as stirring, such as with a mixer or with a stirring bar, which may be a magnetic stirring bar, or the use of boiling stones. A baffle may additionally be present. In a particular embodiment, the heterogeneous catalyst may fulfil the function of boiling stones.

In the process according to the present invention the w/w ratio of heterogeneous catalyst to FDCA is preferably in the range from 100:1 to 1:100, preferably 10:1 to 1:10, and most preferably approximately 1:1. Thus, the present invention allows for use of lower amounts of catalyst than have previously been used in other esterification reactions of aromatic carboxylic acids (Palani et al., J. Mol. Catal., 245(2006) 101-105) .

It is further preferred that the initial concentration of FDCA is in the range from 0.1 to 10 wt.-%, preferably 1 to 5 wt.-%, with respect to the entire reaction mixture, i.e. the entire reaction mixture being 100 wt.-%. The entire reaction mixture is the mixture which comprises the FDCA, the alcohol and the catalyst. The reaction mixture is however not limited to these constituents, as further components such as a solvent as described below and/or a water adsorber as described below and the like may optionally be further constituents comprised in the reaction mixture.

Any alcohol or mixture of alcohols may be used which is suitable to obtain the FDCA ester according to formula I:

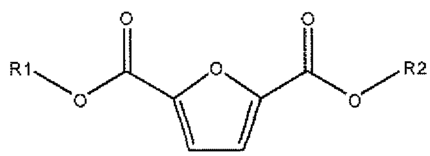

Formula I

wherein R1 is selected from the group consisting of alkyl, aryl, H; and wherein R2 is selected from alkyl, aryl, and wherein the alkyl or aryl moieties may be the same or different and may optionally be substituted. Alkyl is an alkane missing one H; A preferred alkyl within this invention has between one and ten carbon atoms, but preferably between one and four carbon atoms. Aryl refers to any functional group derived from a simple aromatic ring, such as phenyl, thienyl, indolyl and the like. A preferred aryl has between five and ten non-H atoms. If the alkyl and/or aryl is substituted, then preferred substituent(s) may be selected from heteroatom-comprising groups and more preferably from halogenyl. However, unsubstituted alkyl and aryl are even more preferred than substituted alkyl and aryl, unsubstituted alkyl having one to four carbon atoms being most preferred. The alkyl may be cyclic, branched or linear, although linear is preferred. The most preferred alcohol is characterized in being unsubstituted, having one OH group and one to four carbon atoms. That is, it is preferably selected from methanol, ethanol, 1-propanol, 1-butanol and isobutanol. Preferably one alcohol or a mixture of two alcohols is used, but even more preferably, only one alcohol is used. If only one alcohol is used, it is preferably selected as such that the main product of the process according to the invention is selectable from di-methyl ester, di-ethyl ester, di-propyl ester, di-butyl ester and di-isobutyl ester; i.e. the alcohol to be used is the one selected from methanol, ethanol, propanol, butanol and isobutanol, such as in Example 1 or Example 2 below. Mono-and di-esterification of FDCA are comprised in this invention, although di-esterification, i.e. esterification of both carboxyl groups of FDCA is preferred over mono-esterification, i.e. esterification of only one carboxyl group of FDCA. However, the authors of the present invention further noted that in contrary to the study by Palani et al. (wherein only di-esterification of the aromatic carboxylic acid terephthalic acid was reported), mono-esters of FDCA are obtainable with the method of the present invention. The respective mono-esters were obtained as intermediate products upon production of the di-esters. Hence, the present invention provides the further advantage that mono-esters of FDCA are obtainable.

In a further preferred embodiment, the esterification reaction is combined with in-situ separation of volatile compounds. Such a volatile compound is preferably water. Preferred reactor types that allow for in-situ separation of such volatile compounds are reactive distillation devices, reactive rectification devices or membrane reactors. Thus, the water emerging from the condensation of FDCA with the respective alcohol may be removed from the reaction mixture. Any process for water removal known to the skilled person may be applied. Particularly useful but by no means limiting are azeotropic distillation, adsorption (such as adsorption to a molecular sieve) and gas purging. Thus, a means for water removal may be present in the reaction chamber. The preferred mode of water removal is gas purging; hence a purge gas is preferably present in the process according to the invention; the purge gas is preferably an inert gas, i.e. a gas that is non-reactive with the educts, products, intermediates and apparatus of the process according to the present invention. Known and useful inert gases are nitrogen, carbon dioxide, and all noble gases, such as neon and argon. Particularly preferred is a purge gas that is or comprises nitrogen gas, such as in Example 1 below. One main purpose of the purge gas is removal of water that is formed in the esterification process. In the embodiment of carrying out the reaction in the presence of a purge gas, the reaction chamber is further characterized by comprising at least the necessary valves/connections, i.e. at least two valves/connections for inlet and outlet of the purge gas.

Thus, water may be removed from the purge gas after exit from the reaction chamber, such as by condensation, adsorption. The purge gas is preferably recycled. That means, it is depleted of volatile water prior to recycling into the reaction chamber.

During the process according to the present invention, the pressure in the reaction chamber can preferably reach a maximal pressure of 1 bar or more, preferably 3 bar or more and more preferably 5 bar or more. Pressures of up to 25 bar or more, such as shown in example 3, are possible. The pressure may be achieved by exerting external pressure and/or through autogenous reactor pressure, such as in Example 3 below. The skilled person will chose a reaction chamber which is suitable, i.e. stable, at the respective pressure.

After completion of the reaction, the catalysts may be separated from the reaction mixture. Any method suitable for separation of solid particles from a solution is suitable, such as, but not limited to, filtration, sedimentation, centrifugation and the like. Centrifugation is most preferred. The catalyst may optionally be further purified, for example by washing with an organic solvent such as with acetone or with an alcohol. In any case, the catalyst may be recycled, i.e. used as catalyst for further esterification reactions. The mixture that has been separated from the catalyst after the reaction comprises the ester according to the present invention. Typically, this solution also comprises alcohol that was not esterified, and possibly also comprises free FDCA, i.e. FDCA that was not esterified, although, as described above, the amounts of free FDCA are preferably very low, in line with the high yields that can be achieved by the process according to the present invention.

The FDCA ester(s) can be obtained or purified by any method known to the person skilled in the art, without limiting the process of this invention by the method of purification. Preferably, this is done after the separation of the catalyst from the reaction mixture. As an example, one or more of the following processes may be applied: removal of the remaining alcohol and/or the solvent by distillation, precipitation of the ester, re-crystallisation of the ester, chromatography and the like. Precipitation is preferred, and precipitation upon addition of water is most preferred.

In a preferred embodiment, the reaction is carried out in continuous or semi-continuous operation mode. For this purpose, a suitable reactor as known to the person skilled in the art, such as for example a stirred tank reactor or a trickle-bed reactor, is used. In continuous or semi-continuous operation mode, a shape formed catalyst is preferably present inside the reactor vessel.

## Best mode of carrying out the invention

A batch-wise operated stirred tank reactor is charged with alcohol, preferred ethanol or 1-butanol, and FDCA. The catalyst is added, which is preferably a silica-alumina with a $Al_2O_3/SiO_2$ ratio from 0 to 1000, especially preferred a silica-alumina with a $Al_2O_3/SiO_2$ ratio from 0.5 to 5, most preferably used in powder form. The preferred initial FDCA concentration is in the range of 0.1 to 10 wt.-%, especially preferred in the range of 1 to 5 wt-% with respect to the entire reaction mixture. The catalyst amount, relating to the initial amount of FDCA, preferably varies between 10 to 200 wt.-%, especially preferred between 50 to 100 wt.-%. The slurry is heated to the desired reaction temperature. The preferred reaction temperature is 100 to 500°C, especially preferred 200 to 300°C. After the preferable reaction time in the range of 60 to 600 min, especially preferred 180 to 300 min, the reactor is cooled down and the catalyst is removed preferably by filtration, especially preferred by centrifugation, from the product solution and reused. Subsequently, the FDCA-ester is separated from the remaining alcohol preferable by precipitation with water, especially preferred by evaporation of the alcohol.

It is preferred that the reactor, which is preferably a stirred tank reactor or a trickle-bed reactor, is used in continuous or semi-continuous operation mode. For this purpose, a shape formed catalyst is deposited inside the reactor vessel.

It is further preferred that the esterification reaction is combined with in-situ separation of volatile compounds,

preferable water. Preferred reactor types for this purpose are reactive distillation devices, reactive rectification devices or membrane reactors.

**Calculation of reaction characteristics**

For this invention, such as in the description and in the examples below, following reaction characteristics are used:

$$X(FDCA)=\frac{n(FDCA,0)-n(FDCA)}{n(FDCA,0)}\cdot 100\%$$

$$S(i)=\frac{n(i)}{n(FDCA,0)-n(FDCA)}\cdot 100\%$$

$$Y(i)=\frac{n(i)}{n(FDCA,0)}\cdot 100\%$$

| | |
|---|---|
| X(FDCA): | conversion of FDCA [%] |
| S(i): | molar selectivity of FDCA-ester i [%] |
| Y(i): | molar yield of FDCA-ester i [%] |
| n(FDCA,0): | initial amount of FDCA [mol] |
| n(FDCA): | remaining amount of FDCA [mol] |
| n(i): | produced amount of FDCA-ester i [mol]. |

**Examples**

The method of this invention for producing FDCA-esters and is described by the following examples, which are provided for illustration and are not to be construed as limiting the invention.

***Example 1***

A stirred tank reactor (total volume 100 mL) was charged with 60 mL 1-butanol, 0.49 g FDCA and 0.25 g catalyst. The reactor was purged with nitrogen and heated to 200°C giving an autogenous reactor pressure of up to 10 bar. The reaction time measurement started when the temperature was reached. While running the experiment, the agitation speed was kept constant at 800 rpm.

After t=180 min, the reactor was cooled down, the catalyst was filtrated and the reaction solution was analyzed by high-performance liquid chromatography (HPLC).

All the catalysts tested (Süd-Chemie AG: DSY, T-4480, NH4-MOR14, T-4534, Tonsil Supreme 112 FF; Sasol Germany GmbH: Puralox SBa-210, Siralox 5/330, Siralox 30/350) were used in powder form. In case of a shape formed catalyst, it was crushed and sieved before use.

***Table 1:*** Tested catalysts and experimental results for *FDCA-dibutylester synthesis* at 200°C

| sample ID | catalyst type | X (FDCA) [%] | S (FDCA-dibutylester) [%] | Y (FDCA-dibutylester) [%] |
|---|---|---|---|---|
| Puralox SBa-210 | alumina | 95 | 28 | 26 |

(continued)

| sample ID | catalyst type | X (FDCA) [%] | S (FDCA-dibutylester) [%] | Y (FDCA-dibutylester) [%] |
|---|---|---|---|---|
| DSY | zeolite type FAU ($SiO_2$:$Al_2O_3$=12) | 89 | 36 | 32 |
| Siralox 5/330 | silica-alumina ($Al_2O_3$: $SiO_2$= 95:5) | >99 | 35 | 35 |
| Siralox 30/350 | silica-alumina ($Al_2O_3$: $SiO_2$= 70:30) | >99 | 45 | 45 |
| T-4480 | zeolite type MFI, H-form, ($SiO_2$: $Al_2O_3$=90) , $Al_2O_3$ bound extrudates | 90 | 30 | 27 |
| NH4- MOR14 | zeolite type MOR, H-form, ($SiO_2$: $Al_2O_3$=14) | 68 | 28 | 19 |
| T-4534 | zeolite type BEA, H-form, ($SiO_2$: $Al_2O_3$=25) | 87 | 27 | 24 |
| Tonsil Supreme 112 FF | acid activated clay | 91 | 33 | 30 |

***Example 2***

FDCA was esterified with 1-butanol in the same manner as shown in example 1, except that the reactor was heated to 250°C.

***Table 2:*** Tested catalysts and *experimental* results for *FDCA-dibutylester synthesis at 250°C*

| sample ID | catalyst type | X(FDCA) [%] | S(FDCA-dibutylester) [%] | Y(FDCA-dibutylester) [%] |
|---|---|---|---|---|
| Puralox SBa-210 | alumina | >99 | 70 | 70 |
| DSY | zeolite type FAU ($SiO_2$:$Al_2O_3$=12) | >99 | 81 | 81 |
| Siralox 5/330 | silica-alumina ($Al_2O_3$: $SiO_2$= 95:5) | >99 | 77 | 77 |
| Siralox 30/350 | silica-alumina ($Al_2O_3$: $SiO_2$= 70:30) | >99 | >99 | >99 |
| T-4480 | zeolite type MFI, H-form, ($SiO_2$: $Al_2O_3$=90) $Al_2O_3$ bound extrudates | >99 | 58 | 58 |
| NH4- MOR14 | zeolite type MOR, H-form, ($SiO_2$: $Al_2O_3$=14) | 94 | 57 | 54 |
| T-4534 | zeolite type BEA, H-form, ($SiO_2$: $Al_2O_3$=25) | 96 | 56 | 54 |
| Tonsil Supreme 112 FF | acid activated clay | 97 | 79 | 77 |

***Example 3***

A stirred tank reactor (total volume 100 mL) was charged with 60 mL 1-butanol, 0.49 g FDCA and Siralox 30/350 in powder form (silica-alumina, $Al_2O_3$:$SiO_2$=70:30, Sasol Germany GmbH). The reactor was purged with nitrogen and heated to the desired temperature giving an autogenous reactor pressure of up to 25 bar. The reaction time measurement started upon switching-on the reactor heating. While running the experiment, the agitation speed was kept constant at 800 rpm.

After the desired reaction time, the reactor was cooled down, the catalyst was filtrated and the reaction solution was analyzed by HPLC. The catalyst amount, the reaction temperature and the reaction time were varied as shown in table 3.

***Table 3:*** *Experimental* results *for FDCA-dibutylester synthesis*

| reaction temperature [°C] | reaction time [min] | catalyst, amount [g] | X(FDCA) [%] | S(FDCA-dibutylester) [%] | Y(FDCA-dibutylester) [%] |
|---|---|---|---|---|---|
| 194 | 180 | 0,25 | 98 | 30 | 30 |
| 200 | 90 | 0,12 | 48 | 10 | 5 |
| 200 | 90 | 0,37 | 88 | 16 | 15 |
| 200 | 270 | 0,12 | >99 | 40 | 39 |
| 200 | 270 | 0,37 | >99 | 95 | 95 |
| 220 | 64 | 0,25 | 95 | 21 | 20 |
| 220 | 296 | 0,25 | >99 | >99 | >99 |
| 220 | 180 | 0,09 | >99 | 57 | 57 |
| 220 | 180 | 0,40 | >99 | 92 | 92 |
| 220 | 180 | 0,25 | >99 | 80 | 80 |
| 240 | 90 | 0,12 | >99 | 58 | 58 |
| 240 | 90 | 0,37 | >99 | 96 | 96 |
| 240 | 270 | 0,12 | >99 | 86 | 86 |
| 240 | 270 | 0,37 | >99 | 98 | 98 |
| 246 | 180 | 0,25 | >99 | >99 | >99 |

### *Example 4*

FDCA was esterified in the same manner as shown in example 3, except that ethanol was used instead of 1-butanol.

***Table 4:*** *Experimental results f*or *FDCA-diethylester synthesis*

| reaction temperature [°C] | reaction time [min] | catalyst amount [g] | X(FDCA) [%] | S(FDCA-diethylester) [%] | Y(FDCA-diethylester) [%] |
|---|---|---|---|---|---|
| 194 | 180 | 0,25 | 96 | 28 | 27 |
| 200 | 90 | 0,12 | 49 | 9 | 4 |
| 200 | 90 | 0,37 | 93 | 22 | 21 |
| 200 | 270 | 0,12 | 98 | 39 | 38 |
| 200 | 270 | 0,37 | >99 | 77 | 76 |
| 220 | 64 | 0,25 | 78 | 16 | 14 |
| 220 | 296 | 0,25 | >99 | 95 | 94 |
| 220 | 180 | 0,09 | 99 | 59 | 58 |
| 220 | 180 | 0,40 | >99 | 96 | 96 |
| 220 | 180 | 0,25 | >99 | 95 | 95 |

(continued)

| reaction temperature [°C] | reaction time [min] | catalyst amount [g] | X(FDCA) [%] | S(FDCA-diethylester) [%] | Y(FDCA-diethylester) [%] |
|---|---|---|---|---|---|
| 240 | 90 | 0,12 | 92 | 28 | 26 |
| 240 | 90 | 0,37 | >99 | 85 | 84 |
| 240 | 270 | 0,12 | >99 | 76 | 75 |
| 240 | 270 | 0,37 | >99 | 93 | 93 |
| 246 | 180 | 0,25 | >99 | 92 | 92 |

*Example 5*

A stirred tank reactor (total volume 100 mL) was charged with 30 mL 1-butanol, FDCA and Siralox 30/350 in powder form (silica-alumina, $Al_2O_3$:$SiO_2$=70:30, Sasol Germany GmbH). The reactor was purged with nitrogen and heated to 220°C giving an autogenous reactor pressure of up to 15 bar. The reaction time measurement started upon switching-on the reactor heating. While running the experiment, the agitation speed was kept constant at 800 rpm.

After t=240 min, the reactor was cooled down, the catalyst was filtrated and the reaction solution was analyzed by high-performance liquid chromatography (HPLC).

The initial amount of FDCA and the catalyst amount were varied as shown in table 5.

***Table 5:*** *E*xperimental *r*esults *for FDCA-dibutylester synthesis*

| initial amount FDCA [g] | catalyst amount [g] | X(FDCA) [%] | S(FDCA-dibutylester) [%] | Y(FDCA-dibutylester) [%] |
|---|---|---|---|---|
| 0,25 | 0,25 | >99 | >99 | >99 |
| 0,75 | 0,75 | >99 | >99 | >99 |
| 1,28 | 1,28 | >99 | 95 | 95 |
| 2,70 | 2,70 | >99 | 87 | 87 |

*Example 6*

FDCA was esterified in the same manner as shown in example 5, except that ethanol was used instead of 1-butanol.

***Table 6:*** *Experimental results for FDCA-diethylester* synthesis

| initial amount FDCA [g] | catalyst amount [g] | X(FDCA) [%] | S(FDCA-diethylester) [%] | Y(FDCA-diethylester) [%] |
|---|---|---|---|---|
| 0,24 | 0,24 | >99 | 97 | 97 |
| 0,73 | 0,73 | >99 | 93 | 93 |
| 1,25 | 1,25 | >99 | 94 | 94 |
| 2,63 | 2,63 | >99 | 86 | 86 |

## Claims

1. Process for manufacturing esters of 2,5-furandicarboxylic acid by reacting 2,5-furandicarboxylic acid with one or more alcohols in the presence of a heterogeneous catalyst.

2. Process according to claim 1, wherein the catalyst is a Brönsted and/or Lewis acid catalyst.

3. Process according to any one of claims 1 or 2, wherein the heterogeneous catalyst is selected from the group consisting of

a) silica-based materials
b) clays
c) aluminas
d) ion exchange resins
e) metal oxides
f) heteropoly acids
g) hydroxyapatite.

4. Process according to claim 3, wherein the heterogeneous catalyst is a silica-based material selected from the group consisting of

a1) amorphous silica-aluminas
a2) zeolites
a3) mesoporous molecular sieves.

5. Process according to claim 4, wherein the catalyst is amorphous silica-alumina, preferably with an $Al_2O_3/SiO_2$ ratio from 0.2 to 20, more preferably with an $Al_2O_3/SiO_2$ ratio from 1.0 to 3.0, and most preferably with an $Al_2O_3/SiO_2$ ratio from 2.0 to 2.5.

6. Process according to claim 4, wherein the heterogeneous catalyst is a zeolite, preferably of the type FAU, MOR, MFI, LTA or BEA, and especially preferred of the type FAU with an $SiO_2/Al_2O_3$ ratio from 2 to 100.

7. Process according to claim 4, wherein the heterogeneous catalyst is a mesoporous molecular sieve, preferably a metal-doped mesoporous molecular sieve, and more preferably an aluminium-doped mesoporous molecular sieve type MCM-41 with a Si/Al ratio from 5 to 200.

8. Process according to any one of the preceding claims, wherein the catalyst is in the form of particles, preferably in the form of powder.

9. Process according to any one of the preceding claims, wherein the reaction occurs in a reaction chamber which is heated to a temperature of 100 °C or more, preferably to a temperature of 160 °C or more, more preferably to a temperature of 200 °C to 350 °C, and more preferably to a temperature of 250 °C to 300 °C.

10. Process according to any one of the preceding claims, wherein the heating according to claim 9 occurs for 60 min or more, preferably for 60 to 360 min, more preferably for 120 to 300 min.

11. Process according to any one of the preceding claims, wherein the reaction mixture is agitated.

12. Process according to any one of the preceding claims, wherein the w/w ratio of heterogeneous catalyst to FDCA is in the range from 100:1 to 1:100, preferably 10:1 to 1:10, and most preferably approximately 1:1.

13. Process according to any one of the preceding claims, wherein the initial concentration of FDCA is in the range from 0.1 to 10 wt.-%, preferably 1 to 5 wt.-%.

14. Process according to any one of the preceding claims, wherein the one or more alcohol is aliphatic alcohol, preferably monovalent aliphatic alcohol.

15. Process according to claim 14, wherein the one or more aliphatic alcohol comprises or comprise one to four carbon atoms, and is or are preferably selected from methanol, ethanol, 1-propanol, 1-butanol and iso-butanol.

16. Process to any one of claims 14 or 15, wherein one alcohol is used, and wherein the main product is preferably selected from di-methyl ester, di-ethyl ester, di-propyl ester, di-butyl ester and di-isobutyl ester.

17. Process according to any one of the preceding claims wherein the reaction occurs in a chamber in presence of a purge gas, preferentially nitrogen.

**18.** Process according to any one of claims 9 to 17, wherein the pressure in the reaction chamber reaches a maximal pressure of 1 bar or more, preferably 3 bar or more and more preferably 5 bar or more.

Europäisches Patentamt
European Patent Office
Office européen des brevets

# EUROPEAN SEARCH REPORT

Application Number
EP 11 15 2507

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 7 385 081 B1 (GONG WILLIAM H [US]) 10 June 2008 (2008-06-10) * column 5, lines 16-21 * ----- | 1-18 | INV. C07D307/68 |
| Y | WO 2010/077133 A1 (FURANIX TECHNOLOGIES B V [NL]; SIPOS LASZLO [NL]) 8 July 2010 (2010-07-08) * page 10, preparation of dimethyl-2,5-furandicarboxylate * ----- | 1-18 | |
| Y | CHAKRABORTI A K ET AL: "Protic acid immobilized on solid support as an extremely efficient recyclable catalyst system for a direct and atom economical esterification of carboxylic acids with alcohols", JOURNAL OF ORGANIC CHEMISTRY 2009 AMERICAN CHEMICAL SOCIETY USA LNKD- DOI:10.1021/JO900614S, vol. 74, no. 16, 2009, pages 5967-5974, XP002634888, ISSN: 0022-3263 * the whole document * ----- | 1-18 | TECHNICAL FIELDS SEARCHED (IPC): C07D |
| Y | YADAV G D ET AL: "Heterogeneous catalysis in esterification reactions: preparation of phenethyl acetate and cyclohexyl acetate by using a variety of solid acidic catalysts", INDUSTRIAL & ENGINEERING CHEMISTRY RESEARCH, AMERICAN CHEMICAL SOCIETY, US, vol. 33, no. 9, 1 January 1994 (1994-01-01), pages 2198-2208, XP009124575, ISSN: 0888-5885, DOI: DOI:10.1021/IE00033A025 * page 2198, second column, And Table 3 * ----- -/-- | 1-18 | |

The present search report has been drawn up for all claims

1

| Place of search | Date of completion of the search | Examiner |
|---|---|---|
| Munich | 2 May 2011 | Sahagún Krause, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 15 2507

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/097723 A2 (DU PONT [US]; MANZER LEO ERNEST [US]) 20 October 2005 (2005-10-20) * claims 1 and 2 * ----- | 1-18 | |
| Y | WO 2004/099115 A1 (COPPE UFRJ [BR]; ARANDA DONATO ALEXANDRE GOMES [BR]; FAGUNDES MARCOS V) 18 November 2004 (2004-11-18) * page 9, line 16-page 10, line 4 page 10, line 22-24 * ----- | 1-18 | |
| Y,D | LEWKOWSKI, J. ET AL.: "Convenient Synthesis of Furan-2,5-dicarboxylic Acid and ItsDerivatives", POLISH JOURNAL OF CHEMISTRY, vol. 75, 2001, pages 1943-1946, XP009147732, * Scheme 1, page1944 * ----- | 1-18 | |
| | | | TECHNICAL FIELDS SEARCHED (IPC) |

1

The present search report has been drawn up for all claims

| Place of search | Date of completion of the search | Examiner |
|---|---|---|
| Munich | 2 May 2011 | Sahagún Krause, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 15 2507

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2011

| Patent document cited in search report | | Publication date | Patent family member(s) | | | Publication date |
|---|---|---|---|---|---|---|
| US 7385081 | B1 | 10-06-2008 | AR | 071044 | A1 | 26-05-2010 |
| | | | CA | 2705425 | A1 | 22-05-2009 |
| | | | CN | 101868441 | A | 20-10-2010 |
| | | | EP | 2220021 | A1 | 25-08-2010 |
| | | | JP | 2011503187 | T | 27-01-2011 |
| | | | KR | 20100096152 | A | 01-09-2010 |
| | | | US | 2009124829 | A1 | 14-05-2009 |
| | | | WO | 2009064515 | A1 | 22-05-2009 |
| WO 2010077133 | A1 | 08-07-2010 | NONE | | | |
| WO 2005097723 | A2 | 20-10-2005 | EP | 1737810 | A2 | 03-01-2007 |
| WO 2004099115 | A1 | 18-11-2004 | BR | 0301254 | A | 04-10-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2723946
- US 2673860 A
- JP 2008291244 A


### Non-patent literature cited in the description


- Esterification: Methods, Reactions, and Applications. WILEY-VCH Verlag GmbH & Co. KGaA, 2010, 6 ff
- **TAGUCHI et al.** *Chemistry Letters,* 2008, vol. 37, 50
- **LEWKOWSKI.** *Polish Journal of Chemistry,* 2001, vol. 75, 1943
- **CASANOVA et al.** *Journal of Catalysis,* 2009, vol. 265, 109
- Hydrolysis and formation of esters of organic acids. **KIRBY.** Comprehensive Chemical Kinetics, Vol. 10 Ester Formation, Hydrolysis and Related Reactions. Elsevier, 1972, vol. 10, 130 ff
- **T. WERPY ; G. PETERSEN.** Top Value Added Chemicals from Biomass. Volume I - Results of Screening for Potential Candidates from Sugars and Synthesis Gas. National Renewable Energy Laboratory (NREL, 2004, vol. I
- **KOLDITZ.** Anorganikum. Deutscher Verlag, 1970, 423
- **PALANI et al.** *J. Mol. Catal.,* 2006, vol. 245, 101-105